# EUROPEAN PATENT APPLICATION

(11) **EP 4 656 140 A1**
(43) Date of publication of application: **03.12.2025**
(21) Application number: 25174398.5
(22) Date of filing: 06.05.2025
(51) Int. Cl.: A61B 8/08, A61B 8/14, A61B 8/00

(54) **MATERNAL MONITORING SYSTEMS AND METHODS**

(30) Priority: 29.05.2024 US 202418677188
(71) Applicant: GE Precision Healthcare LLC, Waukesha, WI 53188 (US)
(72) Inventor: FALK, Steven M., Waukesha, 53188 (US); KAVOORI SETHUMADHAVAN, Nagapriya, Waukesha, 53188 (US); BOGINENI, Kiran, Waukesha, 53188 (US)
(74) Representative: Kilburn & Strode LLP

(57) **Abstract**

An ultrasound system includes a flexible substrate configured to be maintained on an abdomen of a maternal patient, an transducer array disposed on the flexible substrate and configured to acquire scan data, and a control system. The transducer array comprises a plurality of transducer elements spread across a transducer area sized to image an entire uterus of the maternal patient. The control system is configured to operate the transducer array to acquire the scan data for the entire uterus of the maternal patient at predetermined intervals and generate a uterus image of the entire uterus based on the scan data.

## Description

### BACKGROUND

The present disclosure generally relates to medical monitoring and imaging and, in particular, to a system and method for monitoring maternal patients for postpartum hemorrhage (PPH).

Postpartum hemorrhage results in severe vaginal bleeding after childbirth and is one of the leading causes of maternal death. PPH can occur early postpartum (i.e., within the first 24 hours) or late postpartum (i.e., greater than or equal to 24 hours postpartum). The leading cause of PPH is uterine atony (i.e., the failure of the uterus to contract after birth). Trauma such as lacerations of the uterus, cervix, or vagina may also cause PPH. Retained placenta or clots may also cause PPH. Further, various placenta related causes such as abnormal placentation, including Placenta Accreta Spectrum (PAS); pre-existing or acquired coagulopathy; uterine inversion, etc. may cause PPH. Currently, prevention of PPH is attempted by measures such as routine administration of medication and avoiding birth trauma (e.g., via episiotomy). Signs of PPH may include dizziness, faintness, and blurred vision. However, PPH is difficult to predict and can occur in maternal patients without any risk factors.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one aspect of the present disclosure, an ultrasound system includes a flexible substrate configured to be maintained on an abdomen of a maternal patient, an transducer array disposed on the flexible substrate and configured to acquire scan data, and a control system. The transducer array comprises a plurality of transducer elements spread across a transducer area sized to image an entire uterus of the maternal patient. The control system is configured to operate the transducer array to acquire the scan data of the entire uterus of the maternal patient at predetermined intervals and generate a uterus image of the entire uterus based on the scan data.

In one embodiment, the control system is further configured to generate a plurality of uterus images of the entire uterus and to identify an indicator of postpartum hemorrhage (PPH) based on the plurality of uterus images.

In another embodiment, the control system is further configured to compare the plurality of uterus images to a baseline image to identify the indicator of PPH.

In another embodiment, the indicator of PPH is identified based on a change across the plurality of uterus images.

In another embodiment, the indicator of PPH includes identification of at least one of uterine atony, retained placental tissue, or a presence of endometritis based on the plurality of uterus images.

In another embodiment, the control system is configured to control the transducer array to cause different subsets of the transducer elements to sequentially fire for capturing sub-aperture full matrix capture (FMC) data.

In another embodiment, the FMC data from multiple subsets of transducer elements is utilized to generate the uterus image of the entire uterus.

In another embodiment, the flexible substrate is connected to a stretchable mesh configured to fit around a torso of the maternal patient and maintain the transducer array on the abdomen.

In another embodiment, the flexible substrate comprises a patch configured to adhere to the abdomen.

In another embodiment, the transducer array is configured to image the entire uterus of the maternal patient from one location on the maternal abdomen and without being moved, wherein the transducer array includes a width number of elements distributed across a width of the transducer area and a height number of transducer elements distributed across a height of the transducer area, wherein the width of the transducer area is greater than the height of the transducer area.

In another embodiment, the transducer area is configured to image an area inside the patient that is large enough to span the patient's entire uterus. Optionally, the transducer area may have, for example, a width of about 20 cm, a height of about 30 cm, and a depth of about 20cm.

In another aspect of the present disclosure, a method of monitoring a maternal patient for postpartum hemorrhage (PPH) includes, controlling a transducer array of an ultrasound system to acquire scan data for an entire uterus of a maternal patient. The transducer array comprises a plurality of transducer elements on a flexible substrate configured to be maintained on an abdomen of the maternal patient. The plurality of transducer elements are spread across a transducer area sized to image the entire uterus of the maternal patient without moving the transducer array from its position on an exterior surface of the maternal abdomen. The method further includes generating at least one uterus image of the entire uterus based on the scan data and identifying an indicator of postpartum hemorrhage (PPH) based on the at least one uterus image of the entire uterus.

In one embodiment, the method further includes generating a plurality of uterus images of the entire uterus and identifying the indicator of postpartum hemorrhage (PPH) based on the plurality of uterus images.

In another embodiment, the method further includes comparing the plurality of uterus images to a baseline image to identify the indicator of PPH.

In another embodiment, the method further includes determining a change across the plurality of uterus images, wherein the indicator of PPH is identified based on the change.

In another embodiment, the indicator of PPH includes identification of at least one of uterine atony, retained placental tissue, or presence of an endometritis based on the at least one uterus image of the entire uterus.

In another embodiment, the method further includes automatically controlling the transducer array to acquire the scan data of the entire uterus of the maternal patient at fist predetermined intervals and generating the at least one uterus image of the entire uterus at the predetermined intervals to generate a plurality of uterus images, wherein the indicator of PPH is identified based on the plurality of uterus images.

In another embodiment, the method further includes, in response to identifying the indicator of PPH, adjusting the predetermined interval of image acquisition to a second predetermined interval for a subsequent acquisition of the scan data of the entire uterus, wherein the second predetermined interval is shorter than the first predetermined interval.

In another embodiment, controlling the transducer array further comprises activating subsets of transducer elements of the plurality of transducer elements to capture sub-aperture full matrix capture (FMC) data, and the method further includes generating the image of the entire uterus based on the FMC data captured by a plurality of different subsets of transducer elements.

In another embodiment, the transducer array includes a plurality of subsets of transducer elements between a first side and a second side of the transducer area, wherein activating subsets of transducer elements includes activating each subset of the plurality of subsets of transducer elements sequentially between the first side and the second side.

Various other features, objects, and advantages of the invention(s) will be made apparent from the following description taken together with the drawings.

### BRIEF DESCRIPTION OF THE DRAWINGS

The present disclosure is described with reference to the following Figures.
FIG. 1 is an embodiment of a block diagram of an ultrasound system, in accordance with aspects of the present disclosure.
FIGS. 2-4 are schematic diagrams of one embodiment of a transducer array in accordance with aspects of the present disclosure.
FIG. 5A illustrates an exemplary ultrasound system worn on a maternal patient and comprising a belt having the transducer array in FIG. 2 disposed about the abdomen of the maternal patient, in accordance with aspects of the present disclosure.
FIG. 5B is another illustrates another exemplary ultrasound system worn on a maternal patient and comprising a patch having the transducer array in FIG. 2 disposed on the abdomen of a maternal patient, in accordance with aspects of the present disclosure.
FIG. 6 is a schematic diagram of a diagnostic system comprising part of the ultrasound system in accordance with one embodiment of the present disclosure.
FIG. 7 is a diagrammatic representation of a neural network classifier trained for detection of one or more PPH indicators in accordance with one embodiment of the present disclosure.
FIG. 8 is a diagrammatic representation of a construction of an exemplary trained neural network in accordance with the present disclosure.
FIG. 9 is a flow chart of a method for monitoring a maternal patient for postpartum hemorrhage (PPH).
FIG. 10 is another flow chart of a method for monitoring a maternal patient for postpartum hemorrhage (PPH).

### DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

As used herein, the terms controller or module may refer to, be part of, or include an application-specific integrated circuit (ASIC), an electronic circuit, a combinational logic circuit, a field programmable gate array (FPGA), a processor (shared, dedicated, or group) that executes code, or other suitable components that provide the described functionality, or a combination of some or all of the above, such as in a system-on-chip. The terms controller or module may include memory (shared, dedicated, or group) that stores code executed by the processor. The term code, as used herein, may include software, firmware, and/or microcode, and may refer to programs, routines, functions, classes, and/or objects. The term shared, as used above, means that some or all code from multiple modules may be executed using a single (shared) processor. In addition, some or all code to be executed by multiple different processors may be stored by a single (shared) memory. The term group, as used above, means that some or all code comprising part of a single controller or module may be executed using a group of processors. Likewise, some or all code comprising a single controller or module may be stored using a group of memories.

Aspects of the disclosure are described herein in terms of functional and/or logical block components and various processing steps. It should be appreciated that such block components may be realized by any number of hardware, software, and/or firmware components configured to perform the specified functions. For example, an embodiment may employ various integrated circuit components, e.g., memory elements, digital signal processing elements, logic elements, look-up tables, or the like, which may carry out a variety of functions under the control of one or more processors or other control devices. In addition, those skilled in the art will appreciate that the disclosed invention or inventions may be practiced in conjunction with any number of medical devices, including any number of different physiological data acquisition devices, and that the system described herein is merely one example application. The connecting lines shown in the various figures contained herein are intended to represent example functional relationships and/or physical couplings between the various elements. It should be noted that many alternative or additional functional relationships or physical connections may be present in a practical embodiment.

Postpartum hemorrhage (PPH) is a leading cause of maternal death around the world. The inventors have recognized that while risk factors for PPH are known there does not exist a comprehensive system for evaluating multiple risk factors and identifying high risk patients. While the risk factors of uterine atony, endometritis, Placenta Accreta Spectrum (PAS), and retained placental tissue are known, diagnosis of PPH usually occurs after a patient has gone to the hospital with heavy bleeding, that is after PPH has already occurred.

In view of the foregoing, the inventors have endeavored to build a system which proactively monitors maternal patients and identifies patients at a high risk of PPH. In one aspect of the disclosure, an ultrasound system is worn which periodically scans the maternal patient to generate an image of the uterus of the maternal patient. The image of the uterus is then compared to baseline images to identify the presence of a uterine atony, a retained placental tissue, and/or an endometritis. The identification of a risk factor for PPH can alert caregivers and patients to the need for heightened monitoring and awareness of the risk of PPH and enable caregivers to perform early intervention to prevent PPH or lessen its effects.

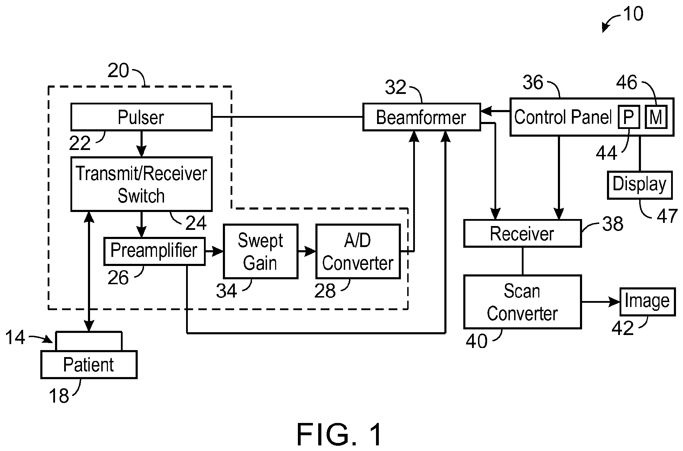

FIG. 1 depicts a high-level view of components of an ultrasound system 10 that may be employed in accordance with the present approach. The illustrated ultrasound system 10 includes an transducer array 14 (e.g., an array of transducer elements arranged together as a single transducer) having transducer elements (e.g., piezoelectric crystals) suitable for contact with a subject or maternal patient 18 during an imaging procedure. The transducer array 14 comprising transducer elements be made by assembling individual transducer crystals or may be a thin film array made by thin film deposition process. Each transducer element in the transducer array 14 may be individually triggerable to transmit and/or receive. The transducer array 14 may be configured as a two-way transducer capable of transmitting ultrasound waves into and receiving such energy from the subject or maternal patient 18. In such an implementation, in the transmission mode the transducer array elements convert electrical energy into ultrasound waves and transmit the ultrasound waves into the maternal patient 18. In reception mode, the transducer array elements convert the ultrasound energy received from the patient 18 (backscattered waves) into electrical signals (or scan data).

Each transducer element in the transducer array 14 is associated with respective transducer circuitry, which may be provided as one or more application specific integrated circuits (ASICs) 20, which (although depicted outside the transducer array 14) may be housed with (e.g., in the same housing as) the transducer array 14. That is, each transducer element in the transducer array 14 is electrically connected to a respective pulser 22, transmit/receive switch 24, preamplifier 26, swept gain 34, and/or analog to digital (A/D) converter 28 provided as part of or on an ASIC 20. In other implementations, this arrangement may be simplified or otherwise changed. For example, components shown in the integrated circuit 20 may be provided upstream or downstream of the depicted arrangement, however, the basic functionality depicted will typically still be provided for each transducer element. In the depicted example, the referenced circuit functions are conceptualized as being implemented on a single ASIC 20 (denoted by dashed line); however, in other embodiments, some or all of these functions may be provided on the same or different integrated circuits. The transducer circuitry may be used to control the switching of the transducer elements. The transducer circuitry may also be used to group the transducer elements into one or more sub-apertures (e.g., in response to control signals from the control system 36).

Also depicted in FIG. 1, a variety of other imaging components are provided to enable image formation with the ultrasound system 10. Specifically, the depicted example of an ultrasound system 10 also includes a beam-former 32, a control system 36, a receiver 38, and a scan converter 40 that cooperate with the transducer circuitry to produce an image or series of images 42 that may be stored and/or displayed to an operator or otherwise processed as discussed herein. The transducer array 14 may communicate the ultrasound data to the beam-former via a wired connection or wireless connection (e.g., via a wireless communication unit that is part of the transducer array that communicates over a wi-fi network, utilizing Bluetooth ^{®} technique, or some other manner). A control system 36 for the ultrasound system 10 includes at least one hardware controller having a processing component 44 (e.g., a microprocessor) and a memory component 46, may be configured to execute stored routines for processing the acquired ultrasound signals to generate meaningful images and/or motion frames of the entire uterus, which may be displayed on a display 47 of the ultrasound system 10. The control system 36 (e.g., in response to the movement of a maternal patient) may utilize one or more algorithms (e.g., stored in memory component 46) to alter where to focus the transducer elements and/or a firing sequence (timing) for triggering the transducer elements. The control system 36 may utilize a trained machine learning model stored in memory component 46, as described below, to compare the generated ultrasound images to baseline ultrasound image(s), for example images of a healthy uterus. The processing component may use the comparison to identify the presence of an indicator of PPH, such as a uterine atony, a retained placental tissue, or a presence of endometritis. When a plurality of ultrasound images has been acquired the control system 36 may then identify the presence of an indicator of PPH by comparing the plurality of uterus images and checking if there has been a change in the uterine image indicating the presence of an indicator of PPH.

Ultrasound information may be processed by other or different mode-related modules (e.g., B-mode, Color Doppler, power Doppler, M-mode, spectral Doppler anatomical M-mode, strain, strain rate, and the like) to form 2D or 3D data sets of image frames and the like. For example, one or more modules may generate B-mode, color Doppler, power Doppler, M-mode, anatomical M-mode, strain, strain rate, spectral Doppler image frames and combinations thereof, and the like. The image frames are stored and timing information indicating a time at which the image frame was acquired in memory may be recorded with each image frame. The modules may include, for example, a scan conversion module to perform scan conversion operations to convert the image frames from Polar to Cartesian coordinates. A video processor module may be provided that reads the image frames from memory and displays the image frames in real time while a procedure is being carried out on a patient. A video processor module may store the image frames in an image memory, from which the images are read and displayed. The ultrasound system 10 shown may comprise a console system, or a portable system, such as a hand-held or laptop-type system.

The ultrasound system 10 may be operable continuously or at predetermined intervals to acquire ultrasound scan data at a frame rate that is suitable for the imaging situation. Typical frame rates may range from 20-120 fps but may be lower or higher. The acquired ultrasound scan data may be displayed on the display 47 at a display-rate that can be the same as the frame rate, or slower or faster. An image buffer may be included for storing processed frames of acquired ultrasound scan data that are not scheduled to be displayed immediately. Preferably, the image buffer is of sufficient capacity to store at least several minutes worth of frames of ultrasound scan data. The frames of ultrasound scan data are stored in a manner to facilitate retrieval thereof according to its order or time of acquisition. The image buffer may be embodied as any known data storage medium. Notably, the frame rate for imaging the uterus may be relatively low since the uterus is not rapidly moving. For example, the frame rate may be one image every few seconds, or even once every few minutes.

The display 47 may be any device capable of communicating visual information to a user. For example, the display 47 may include a liquid crystal display, a light emitting diode (LED) display, and/or any suitable display or displays. The display 47 can be operable to present ultrasound images and/or any suitable information.

Components of the ultrasound system 10 may be implemented in software, hardware, firmware, and/or the like. The various components of the ultrasound system 10 may be communicatively linked. Components of the ultrasound system 10 may be implemented separately and/or integrated in various forms.

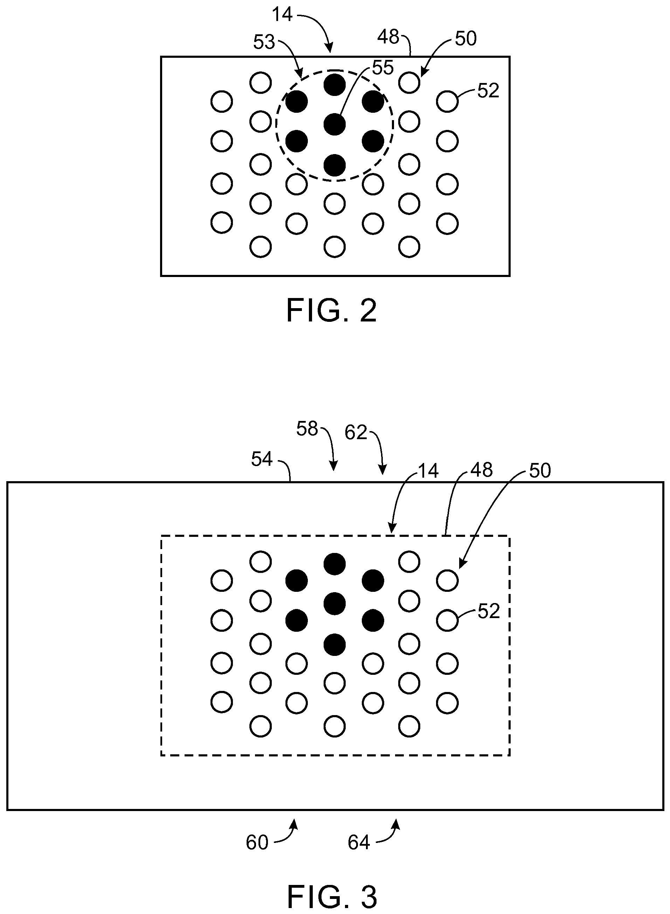

FIG. 2 is a schematic diagram of one embodiment of the transducer array 14 (e.g., cooperating together to form a single ultrasound transducer system). Here, the transducer array 14 includes a flexible substrate 48 and a flexible array 50 formed of transducer elements 52 (e.g., piezoelectric crystals) on or connected together by the flexible substrate 48. The transducer may be made by assembling individual transducer crystals onto the flexible substrate 48 or may be a thin film array made by thin film deposition process. In certain embodiments, the transducer elements 52 may be made of lead zirconate titanate (PZT). In various embodiments, the flexible substrate 48 may be formed of any of various flexible, biocompatible materials, such as silicone or polymers such as polypropylene, polyamide, polycarbonate, or the like. The substrate may also be composed of a biocompatible fabric, such as polyester, and/or organic fibers such as cotton. The flexible substrate 48 may be stretchable, such as to elastically deflect over the maternal abdomen and change shape with the maternal abdomen throughout the birthing process. A variety of different materials may be suitable for such a substrate including ferro-electric polymers from PVDF family of materials.

The number of transducer elements 52 forming the flexible array 50 may vary from 10 to 100 or more transducer elements 52. During operation, the transducer elements 52 function to acquire data for imaging an entire uterus of a maternal patient and/or identify one or more indicators of postpartum hemorrhage (PPH), such as a uterine atony, retained placental tissue, or presence of endometritis. Different subsets of the transducer elements 52 (e.g., 2 or more transducer elements 52 but less than the total number of transducer elements 52 that form the flexible array 50) are triggered or fired sequentially (i.e., to transmit ultrasound waves). Thus, one subset of transducer elements 52 may be fired or triggered, then a different subset of transducer elements 52 may be fired or triggered, and the firing sequence continues until each of the transducer elements 52 have been fired or triggered. In certain embodiments, the data is acquired utilizing sub-aperture full matrix capture (FMC). In certain embodiments, the transducer elements 52 may be sequentially fired or triggered individually (as opposed to with a subset). The different subsets may have the same number of transducer elements 52 or vary in the number of transducer elements 52 making up the subset. All transducer elements 52 of the flexible array 50 receive the ultrasound energy returned from the patient. For example, assume 6 of the transducer elements 52 form a subset 53 of the transducer elements 52 and transducer element 55 of the subset 53 transmits ultrasound waves, then transducer element 55, the rest of the subset 53 of transducer elements 52 and all of the transducer elements 52 outside the subset 53 receive the ultrasound energy returned from the patient. The layout of the transducer elements 52 as depicted is two-dimensional (2D) scaled. This utilization of the transducer elements 52 ensures sufficient depth of field for Doppler measurements to enable measurement of indications of postpartum hemorrhage (PPH). As described in greater detail below, the acquisition of scan data from the sequential firing of the transducer elements enables the localization of indicators of PPH within the uterus.

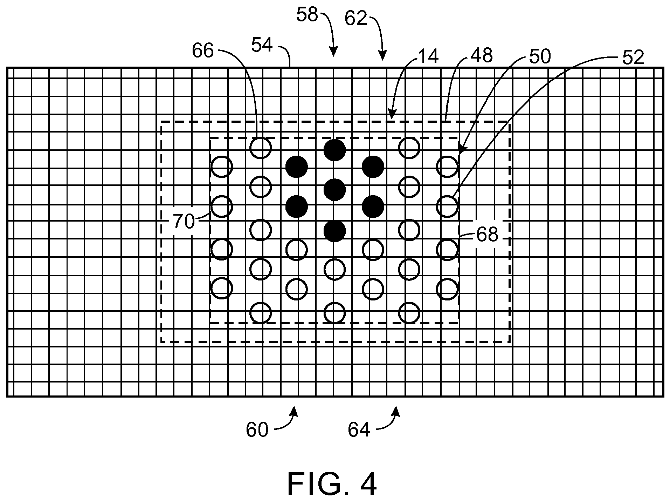

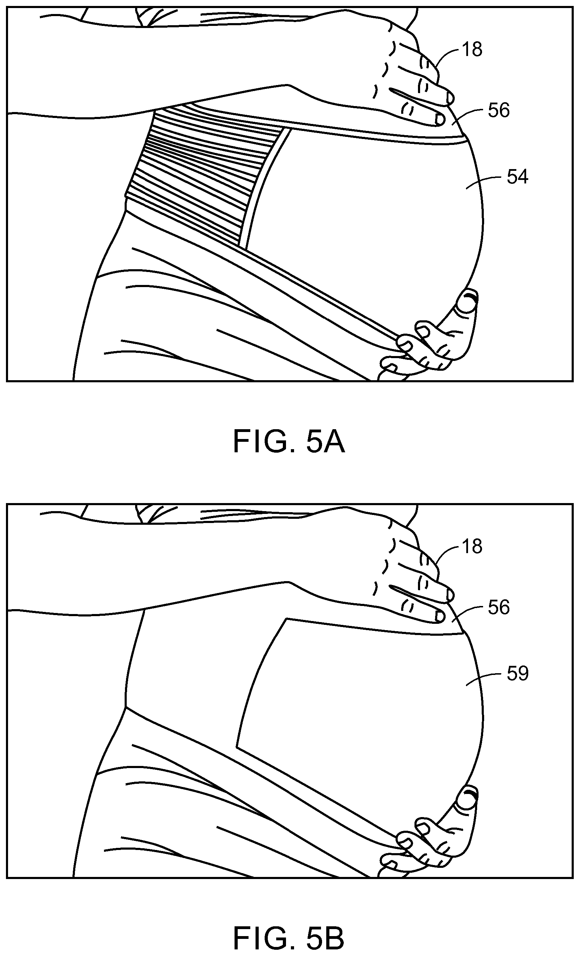

As depicted in FIG. 3, the flexible substrate 48 may be part of a single flexible belt 54 that is configured to be disposed about the abdomen of the patient (e.g., such as a flexible belt 54 in FIG. 5A disposed about an abdomen 56 of a maternal patient 18). The flexible substrate 48 may be part of the flexible belt 54 or coupled to the flexible belt 54. The transducer array 14 is disposed on an inner surface of the belt 54 and oriented so that the transducer elements 52 face the abdomen. The belt 54 may be designed to be comfortable on the patient. For example, the flexible belt 54 may be a flexible belt made of a stretchable mesh material, such as an elastic belt, as shown in FIG. 4 (e.g., similar to the material utilized with postpartum underwear), that is thin, soft, breathable, and cool. The flexible belt 54 may also be stretchy so as to conform to the different sizes of patients. In certain embodiments, the flexible belt 54 may have full openings 58, 60 on respective ends 62, 64 (e.g., superior and inferior ends) to enable the flexible belt 54 to be slid along the patient's body until it is disposed about the abdomen. In certain embodiments, the flexible belt 54 may be adjustable for fit and wrapped around the abdomen of the patient (e.g., similar to abdominal binders) and secured via fasteners (e.g., hook and loop fasteners).

In still other embodiments, the transducer array 14 may be attached to and maintained on the maternal abdomen by other means. For instance, as shown in FIG. 5B, the flexible substrate 48 may be part of a patch 59 adhering to the abdomen 56 of a maternal patient 18. The patch 59 is configured to continue to adhere to the abdomen 56 of the maternal patient 18 as the abdomen 56 changes shape throughout the birthing process. The patch 59 may be comprised of a flexible material upon which the flexible substrate 48 is mounted or otherwise embedded. For example, the patch 59 may comprise a flexible foam material configured to expand and contract to allow the movement of the flexible substrate 48 to conform to the changing shape of the maternal abdomen throughout the birthing process. Altnernatively, the patch may be comprised of the flexible substrate material, and thus be one with the flexible substrate 48, such that the transducer elements 52 are mounted on the substrate material of the patch. The patch 59 is configured to maintain the transducer area 56 against the skin of the maternal abdomen sufficiently to enable the ultrasound imaging by the transducer array 14 of the entire uterus described herein.

The patch 59 may adhere to the skin of the maternal abdomen via a biocompatible adhesive, such as a pressure-sensitive adhesive (PSA) such as made from materials like acrylic, silicone, and rubber. The patch 59 may have adhesive on its outer edges, or on portions thereof, surrounding the transducer area. The patch 59 may have adhesive strategically placed around the transducer area 56 and configured to facilitate the flexing of flexible substrate 48 with the maternal patient's abdomen. For example, the patch 59 may have adhesive on its lateral sides, outside of the transducer area 56 and adjacent to the sides 68 and 70 (see FIG. 4). Alternatively or additionally, the patch 59 may include adhesive at strategic and intermittent locations along the top and bottom edges above and below the transducer area 56, such as centered above and blow the transducer area 56 and/or above and below the lateral sides 68 and 70 of the transducer area 56 so as to maintain the transducer array against the maternal abdomen to enable continued imaging throughout and after the delivery process.

As depicted in FIG. 4 the transducer elements 52 are spread across the transducer area 66. Transducer area 66 is an area on the flexible substrate occupied by the transducer elements and is configured to image an entire uterus of a maternal patient from one location on the skin surface of maternal abdomen and without being moved. As transducer area 66 remains constant while the size of maternal uterus varies from patient to patient, the transducer area 66 is configured such that different sized uteruses may be fully imaged when the transducer array 14 is placed on the maternal patient. Thus transducer area 66 is configured to image an area within the patient that is large enough to span the patient's entire uterus, such as via beam steering, without moving or repositioning the transducer array 14 on the skin surface of the maternal abdomen. Adjusting where to focus the transducer elements 52 and/or changing the firing sequence (e.g., timing) of the transducer elements 52 enables the transducer area 66 to be held constant while the area of the uterus to be sized changes from patient to patient.

Transducer area 66 is the area on the flexible substrate occupied by the plurality of transducer elements 52 and has a first side 68 and an opposite second side 70. For example, the plurality of transducer elements may be assembled as individual transducer crystals or may be a thin film array made by a thin film deposition process. Transducer area 66 exemplarily has a width between the first side 68 and the second side 70 of about 15 cm and a height of about 10 cm, although other combinations of width and height will be apparent to those skilled in the art. In various embodiments, the transducer area may be at least 10 cm wide, such as between 10 cm and 20 cm wide, and at least 5 cm high, such as between 5 cm and 10 cm (or taller, such as a height of 15 cm). In one such embodiment, the transducer area 66 may contain an array of transducer elements 52 that is 512 elements across (distributed across the width) and 64 elements vertical (distributed across the height). In some embodiments, the transducer elements 52 are sequentially fired from the first side to the second side to image the entire uterus. The transducer area 66 may smaller than the area inside the patient that is imaged. The area imaged in the patient may have, for example, a width of about 20 cm, a height of about 30 cm, and a penetration depth of about 20cm, and the transducer array 14 is configured to emit ultrasound to penetrate the abdomen to an appropriate area and depth to image an area inside the patient that spans the entire uterus.

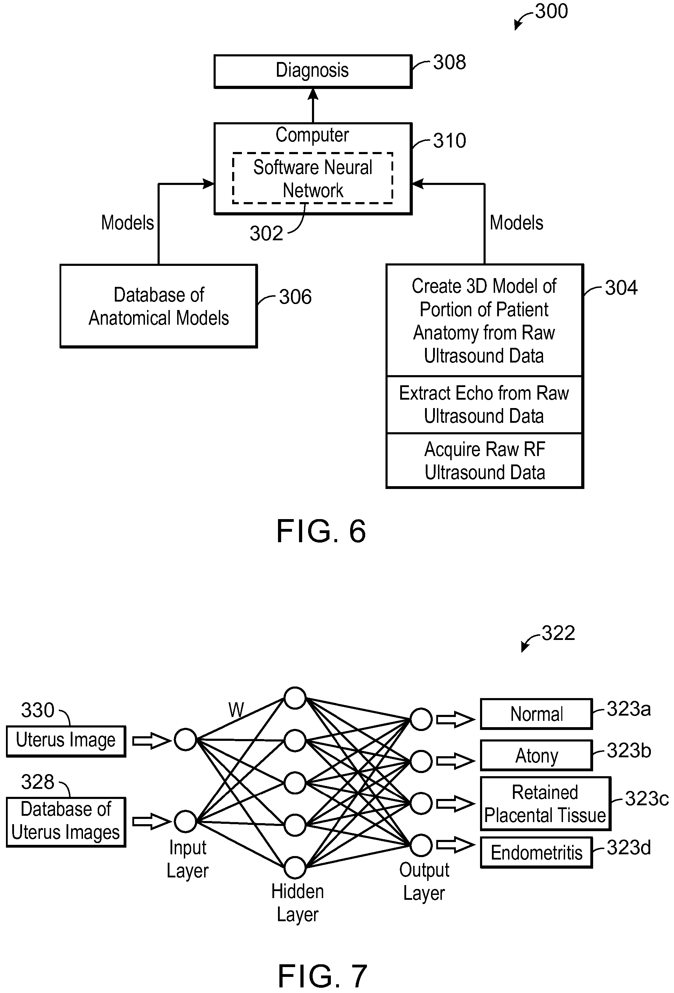

The ultrasound system 10 may be configured such that the ultrasound images are assessed and translated into data usable for assessing PPH, including identifying one or more PPH indicators. In some embodiments, the PPH indicator(s) may be identified by a doctor or other clinician assessing the ultrasound image(s) and manually entering information indicating positive or negative for various PPH factors, such as those listed herein. Alternatively, the ultrasound system 10 may be configured to automatically process the ultrasound images to identify one or more indicators of PPH. FIG. 6 illustrates one embodiment in which, the ultrasound images 42 are send to the computer 310 configured to process the images with software configured to detect indication(s) of PPH. The diagnostic system 300 may include a software based neural network 302, which may be in the form of program code residing in the memory of computer 310. The diagnostic system 300 may operate to identify an indication of PPH such as a uterine atony, an endometritis, or a retained placental tissue. A first module 304 may output a 3-D model of a portion of the patient's anatomy, specifically the uterus of a maternal patient, to the computer 310 for data processing by way of neural network 302. A second module 306 may include a database of anatomical datasets or models and may output one or more of these models to the computer 310 for processing by the neural network 302. That is, the 3-D model may be compared to the database of models by the neural network 302. The neural network 302 may then return a diagnosis based on the comparison. The data processing may provide one or more of a visual output, an audible output, and a diagnosis 308 by way of a suitable visual display, such as the display 47.

FIG. 7 illustrates one embodiment of a neural network classifier 322 having multiple outputs 323a, 323b, 323c, 323d (e.g., binary outputs where each output is either a "1" or a "0" wherein the "1" corresponds to "yes" and the "0" corresponds to "no"). In this neural network classifier, each output 323a, 323b, 323c, 323d represents the response of the neural network 302 to a particular set of inputs containing one or more indicator of PPH. For example, one output 323*a* may represent a normal condition where indicators of PPH are not present, while another output 323b may represent the response for the presence or absence of a uterine atony. In either case, the output state of the respective indicator will be "1" if the indicator is detected as being present and "0" otherwise. Similarly, the neural network 302 may output an appropriate state for other indicators of PPH, such as retained placental tissue 323c or endometritis 323d. The inputs to this neural network classifier are an image of the patient's uterus 330 and a database of uterus images 328 of healthy and diseased uteruses. The neural network 302 and the classifier 322 may be significantly more or less sophisticated, depending on the underlying model of the anatomical feature(s) in question (i.e., the uterus). Alternatively or additionally, other outputs may be include for other indicators of PPH, such as abnormal placentation-e.g.a, Placenta Accreta Spectrum (PAS).

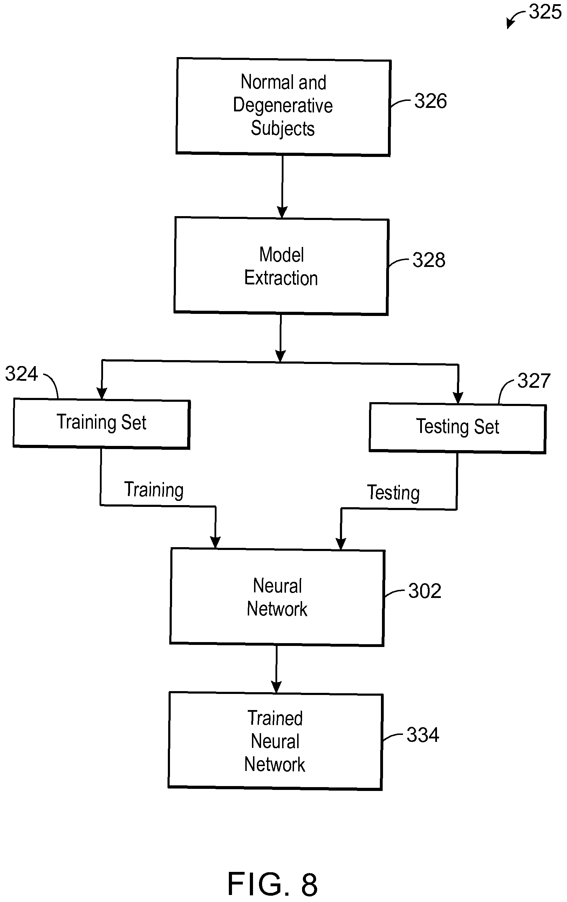

FIG. 8 illustrates one embodiment of a construction 325 of a trained neural network 334 to be utilized for detecting one or more PPH indicators based on ultrasound images acquired from a maternal patient. A group of uterus images stored in a database 328 is formulated (such by the above-described processes or by any process for generating ultrasound images of uteruses) to be used as training and testing data, which includes uterus images from subjects 326, both normal subjects and those exhibiting one or more indicators of PPH. The construction 325 includes formulating a supervised classifier using a labeled training set 324 of uterus images, including normal uterus images and abnormal uterus images containing one or more indicators of PPH. The neural network 302 is trained with the training set 324, wherein each training image consists of data (e.g., 3-D ultrasound models) collected from one or more patients or test subjects and wherein each image in the training set 324 is labeled, including labels as being positive or negative for PPH indicators and whether the patient from which the images were obtained ultimately developed PPH or not. The training set may also include pre-birth images. The neural network 302 is tested using the testing set 327, such as to generate feedback for further training the neural network 302 so as to generate the trained neural network 334.

The flexible belt 54 is designed to be worn throughout and following the birthing process, including during any or all of the antepartum, intrapartum, and postpartum phases of labor. The flexible belt 54 may be donned by the maternal patient in the early stages of labor or before labor has begun. Transducer array 14 then proceeds to image the entire uterus throughout the labor process. Post-labor the flexible belt 54 continues to be worn. The transducer array 14 continues to image the entire uterus and repositionining of the flexible belt 54 is unnecessary. Flexible substrate 48 is configured to flex and adjust its shape as the maternal abdomen changes shape throughout the phases of labor, including postpartum. Flexible susbtrate 48 is configured to attach to the maternal abdomen via flexible belt 54 or patch 59. Flexible substrate 48 is further configured to facilitate the movement and flexion of transducer array 14 such that transducer array 14 remains positioned and substantially stationary on the maternal abdomen ssufficiently such that that it can continue to image the entire maternal uterus throughout the birth process and postpartum, even as the shape of the maternal abdomen flexes and changes.

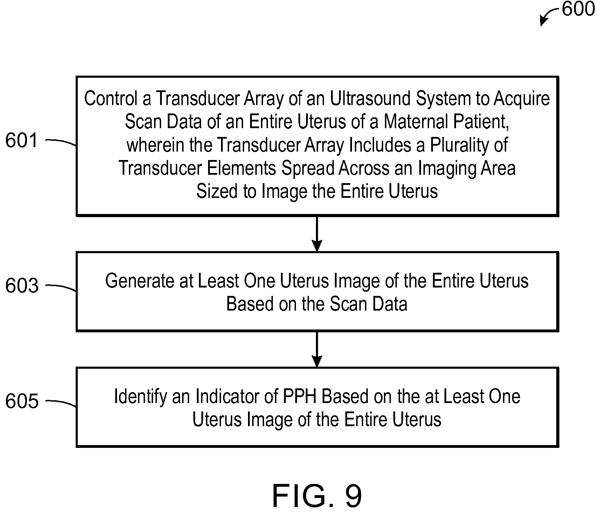

FIG. 9 is a flowchart of a method 600 for monitoring a maternal patient for PPH. At step 601 a transducer array of an ultrasound system is controlled to acquire scan data of an entire uterus of a maternal patient. The transducer array comprises a plurality of transducer elements, for example, transducer elements on a flexible substrate configured to be maintained in a stationary position on an abdomen of the maternal patient to acquire the uterus image. The plurality of transducer elements are spread across a transducer area sized to image the entire uterus of a patient without moving the transducer array. At step 603 at least one uterus image of the entire uterus is generated based on the scan data acquired in step 601. Finally, at step 605, an indicator of PPH-such as uterine atony, retained placental tissue, or presence of endometritis-is identified based on the least one uterus image of the entire uterus.

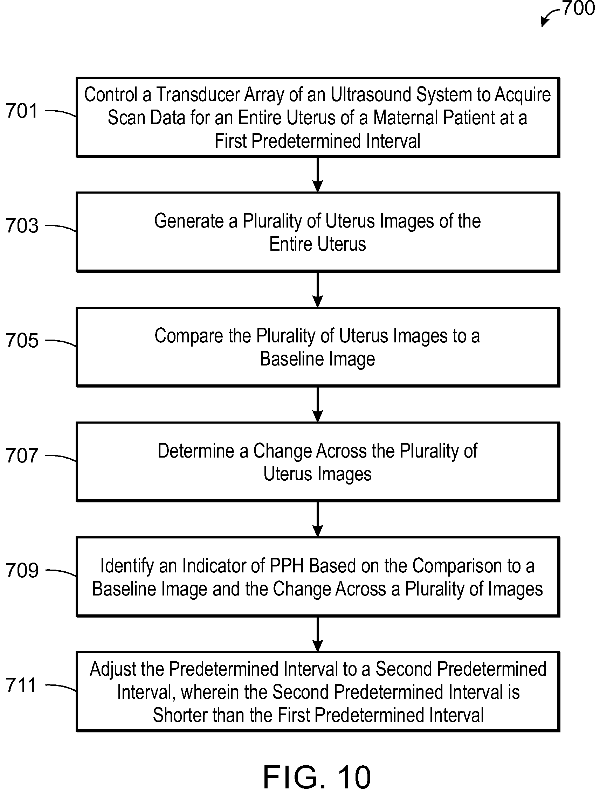

FIG. 10 is a flowchart of a method 700 for monitoring a maternal patient for PPH. At step 701 a transducer array of an ultrasound system is controlled to acquire scan data for an entire uterus of a maternal patient at a first predetermined interval. In some embodiments, controlling the transducer array further comprises activating subsets of transducer elements of the plurality of transducer elements to capture sub-aperture full matrix capture (FMC) data, generating the image of the entire uterus based on the FMC data generated by a plurality of different subsets of transducer elements. In some embodiments, the transducer array includes a plurality of subsets of transducer elements between a first side and a second side of the transducer area and controlling the transducer array further comprises activating subsets of transducer elements sequentially between the first side and the second side.

At step 703 a plurality of uterus images of the entire uterus are generated, such as ultrasound images 42 generated by the ultrasound system 10 as described above. At step 705, the plurality of uterus images are compared to a baseline image, which may be a generalized baseline depicting a healthy uterus or may be a patient-specific baseline, such as an image of the patient's uterus acquired at an earlier time. At step 707 a change is detected across the plurality of uterus images, such as between a first image and one or more later-captured images of the entire uterus. At step 709 an indicator of PPH is identified based on the comparison to a baseline image and the change across a plurality of uterus images. At step 711, the predetermined interval is adjusted to a second predetermined interval, wherein the second predetermined interval is shorter than the first predetermined interval such that the ultrasound imaging of the uterus happens more frequently.

The above-described steps of the processes of FIGS. 9 and 10 are not limited to the order and sequence shown and described in the figures and in various embodiments the steps may be performed in another order. Some of the above steps of the processes of FIGS. 9 or 10 can be executed or performed substantially simultaneously where appropriate or in parallel to reduce latency and processing times.

This written description uses examples to disclose the invention(s), including the best mode, and also to enable any person skilled in the art to make and use the invention(s). Certain terms have been used for brevity, clarity, and understanding. No unnecessary limitations are to be inferred therefrom beyond the requirement of the prior art because such terms are used for descriptive purposes only and are intended to be broadly construed. The patentable scope of the invention(s) is defined by the claims and may include other examples that occur to those skilled in the art based on the entirety of present disclosure. Such other examples are intended to be within the scope of the claims if they have features or structural elements that do not differ from the literal language of the claims, or if they include equivalent features or structural elements with insubstantial differences from the literal languages of the claims.

## Claims

1. An ultrasound system, comprising:
a flexible substrate configured to be maintained on an abdomen of a maternal patient;
an transducer array disposed on the flexible substrate and configured to acquire scan data, wherein the transducer array comprises a plurality of transducer elements spread across a transducer area sized to image an entire uterus of the maternal patient;
a control system comprising at least one hardware controller and configured to:
operate the transducer array to acquire the scan data of the entire uterus of the maternal patient at predetermined intervals; and
generate a uterus image of the entire uterus at the predetermined intervals based on the scan data.

2. The system of claim 1, wherein the control system is further configured to generate a plurality of uterus images of the entire uterus and to identify an indicator of postpartum hemorrhage (PPH) based on the plurality of uterus images.

3. The system of claim 2, wherein the control system is further configured to compare the plurality of uterus images to a baseline image to identify the indicator of PPH.

4. The system of claim 2, wherein the indicator of PPH is identified based on a change across the plurality of uterus images.

5. The system of claim 2, wherein the indicator of PPH includes identification of at least one of uterine atony, retained placental tissue, Placenta Accreta Spectrum, or presence of an endometritis based on the plurality of uterus images.

6. The system of claim 1, wherein the control system is configured to control the transducer array to cause different subsets of the transducer elements to sequentially fire for capturing sub-aperture full matrix capture (FMC) data, and/or wherein FMC data from multiple subsets of transducer elements is utilized to generate the uterus image of the entire uterus.

7. The system of claim 1, wherein the flexible substrate is connected to a stretchable mesh configured to fit around a torso of the maternal patient and maintain the transducer array on the abdomen.

8. The system of claim 1, wherein the flexible substrate comprises a patch configured to adhere to the abdomen.

9. The system of claim 1, wherein the transducer array includes a width number of elements distributed across a width of the transducer area and a height number of transducer elements distributed across a height of the transducer area, wherein the width of the transducer area is greater than the height of the transducer area, and/or wherein the width of the transducer area is at least 15 cm and the height of the transducer area is at least 5 cm.

10. A method of monitoring a maternal patient for postpartum hemorrhage (PPH), the method comprising:
controlling an transducer array of an ultrasound system to acquire scan data for an entire uterus of a maternal patient, wherein the transducer array comprises a plurality of transducer elements on a flexible substrate configured to be maintained on an abdomen of the maternal patient and wherein the plurality of transducer elements are spread across a transducer area sized to image the entire uterus of the maternal patient;
generating at least one uterus image of the entire uterus based on the scan data; and
identifying an indicator of postpartum hemorrhage (PPH) based on the at least one uterus image of the entire uterus.

11. The method of claim 10, further comprising generating a plurality of uterus images of the entire uterus and identifying the indicator of postpartum hemorrhage (PPH) based on the plurality of uterus images, and/or further comprising comparing the plurality of uterus images to a baseline image to identify the indicator of PPH, or determining a change across the plurality of uterus images, wherein the indicator of PPH is identified based on the change.

12. The method of claim 10, wherein the indicator of PPH includes identification of at least one of uterine atony, retained placental tissue, Placenta Accreta Spectrum, or presence of an endometritis based on the at least one uterus image of the entire uterus.

13. The method of claim 10, further comprising automatically controlling the transducer array to acquire the scan data of the entire uterus of the maternal patient at first predetermined intervals and generating the at least one uterus image of the entire uterus at the first predetermined intervals to generate a plurality of uterus images, wherein the indicator of PPH is identified based on the plurality of uterus images, and/or further comprising, in response to identifying the indicator of PPH, automatically controlling the transducer array to acquire the scan data of the entire uterus of the maternal patient at second predetermined intervals for subsequent acquisition of the scan data for the entire uterus, wherein the second predetermined intervals are is shorter than the first predetermined intervals.

14. The method of claim 10, wherein controlling the transducer array further comprises activating subsets of transducer elements of the plurality of transducer elements to capture sub-aperture full matrix capture (FMC) data, and wherein the image of the entire uterus is generated based on the FMC data captured by a plurality of different subsets of transducer elements.

15. The method of claim 10, wherein the transducer array includes a plurality of subsets of transducer elements between a first side and a second side of the transducer area, wherein activating subsets of transducer elements includes activating each subset of the plurality of subsets of transducer elements sequentially between the first side and the second side.
